# EUROPEAN PATENT APPLICATION

(11) **EP 1 864 605 A1**
(43) Date of publication of application: **12.12.2007**
(21) Application number: 06714214.1
(22) Date of filing: 21.02.2006
(51) Int. Cl.: A61B 1/04, H04N 7/18

(54) **SIGNAL PROCESSING DEVICE FOR ELECTRONIC ENDOSCOPE, AND ELECTRONIC ENDOSCOPE DEVICE**

(30) Priority: 29.03.2005 JP 2005095716
(71) Applicant: OLYMPUS MEDICAL SYSTEMS CORP., Shibuya-ku Tokyo 151-0072 (JP)
(72) Inventor: TAKEMURA, Takashi c/o Olympus Medical Systems Corp., Tokyo 151-0072 (JP)
(74) Representative: von Hellfeld, Axel
(86) International application number: PCT/JP2006/303074
(87) International publication number: WO 2006/103846

(57) **Abstract**

An electronic endoscope provided with a plurality of solid-state image pickup devices is connected to the endoscope connection portion of a signal processing apparatus for an electronic endoscope. The signal processing apparatus for an electronic endoscope includes a selecting operation portion for performing the operation to generate a selecting signal to allow the selection of at least one output signal of the plurality of solid-state image pickup devices provided in the electronic endoscope connected to the endoscope connecting portion; a signal processing circuit performing signal processing for the output signal of one solid-state image pickup device selected based on the selecting signal and generating a video signal, and an information switch control portion performing the switching of the information to be displayed in the display device regarding the electronic endoscope corresponding to one selected solid-state image pickup device whose output signal is selected.

## Description

### Technical Field

The present invention relates to a signal processing apparatus for an electronic endoscope and an electronic endoscope apparatus performing signal processing for an electronic endoscope including a plurality of solid-state image pickup devices.

### Background Art

An electronic endoscope whereupon a solid-state image pickup device is provided on the distal end portion of an insertion portion has been in wide use in an endoscopic examination, a procedure by a treatment instrument or the like in the medical fields.

For example, Japanese Patent Application Laid-Open No. 2003-26410 as a precedent discloses that the information on the treatment instrument channel of the electronic endoscope is displayed on the screen of a monitor. By this display, the precedent allows a user to find out an external diameter of the treatment instrument such as forceps fit for use and from which direction the treatment instrument comes out on the endoscopic screen.

However, the above described precedent is unable to appropriately deal with the electronic endoscope mounting two solid-state pickup devices on the distal end portion of the insertion portion.

In applying the above described precedent to the case of the electronic endoscope mounting two solid-state pickup devices, when the use is switched or selected from one solid-state image pickup device used for observation to the other solid-state image pickup device, the position of the distal end opening of the treatment instrument channel changes relative to the switched solid-state image pickup device. Hence, in the precedent, even when the same forceps are used, the direction from which the forceps appear in the observation field of view varies, and becomes different from the information on the displayed treatment instrument channel.

Further, in the precedent mentioned above, when a zoom scale is displayed on the endoscope screen, and when the use between different solid-state image pickup devices is switched, the size thereof shows a different value.

The present invention has been made in view of the above described points, and a subject of the invention is to provide a signal processing apparatus for an electronic endoscope and an electronic endoscope apparatus capable of displaying information appropriately corresponding to a selection when the selection of one solid-state image pickup device actually used in the electronic endoscope mounting a plurality of solid-state image pickup devices is performed.

### Disclosure of the Invention

### Means for Solving the Problem

The signal processing apparatus for an electronic endoscope of the present invention comprises:
an endoscope connecting portion connected with an electronic endoscope provided with at least a plurality of solid-state image pickup devices,
a selecting operation portion for performing an operation to generate a selecting signal to allow the selection of at least one output signal of the plurality of solid-state image pickup devices provided in the electronic endoscope connected to the endoscope connection portion;
signal processing circuit performing signal processing for the output signal of one solid-state image pickup device selected based on the selecting signal and generating a video signal; and
an information switch control portion performing the switching of the information to be displayed in the display device regarding the electronic endoscope, corresponding to one selected solid-state image pick up device whose output signal is selected.

By the above described configuration, the information regarding the electronic endoscope displayed in the display device corresponding to the case of the solid-state image pickup device actually selected by the information switch control portion can be made appropriate.

The electronic endoscope apparatus of the present invention comprises:
an electronic endoscope provided with a plurality of solid-state image pickup devices;
a selection operating portion for performing an operation to generate a selecting signal to allow the selection of at least one output signal of the plurality of solid-state image pickup devices provided in the electronic endoscope;
signal processing circuit performing signal processing for the output signal of one solid-state image pickup device selected based on the selecting signal and generating a video signal; and
an information switch control portion performing the switching of the information to be displayed in the display device regarding the electronic endoscope, corresponding to one selected solid-state image pick up device whose output signal is selected.

By the above described configuration, the information regarding the electronic endoscope displayed in the display device corresponding to the case of the solid-state image pickup device actually selected by the information switch control portion can be made appropriate.

### Brief Description of the Drawings

Fig. 1 is a block diagram showing a whole configuration of an electronic endoscope apparatus comprising a first embodiment of the present invention;
Fig. 2 is a front view of a distal end portion;
Fig. 3 is a view showing a configuration of rotating filters and filter characteristics and the like;
Fig. 4 is a flowchart showing a content of operation in the present embodiment;
Fig. 5A is a view showing a display screen of a monitor at the normal observation mode time; and
Fig. 5B is a view showing a display screen of a monitor at the fluorescent light observation mode time.

### Best Mode for Carrying Out the Invention

Hereinafter, an embodiment of the present invention will be described with reference to the drawings.

### (First Embodiment)

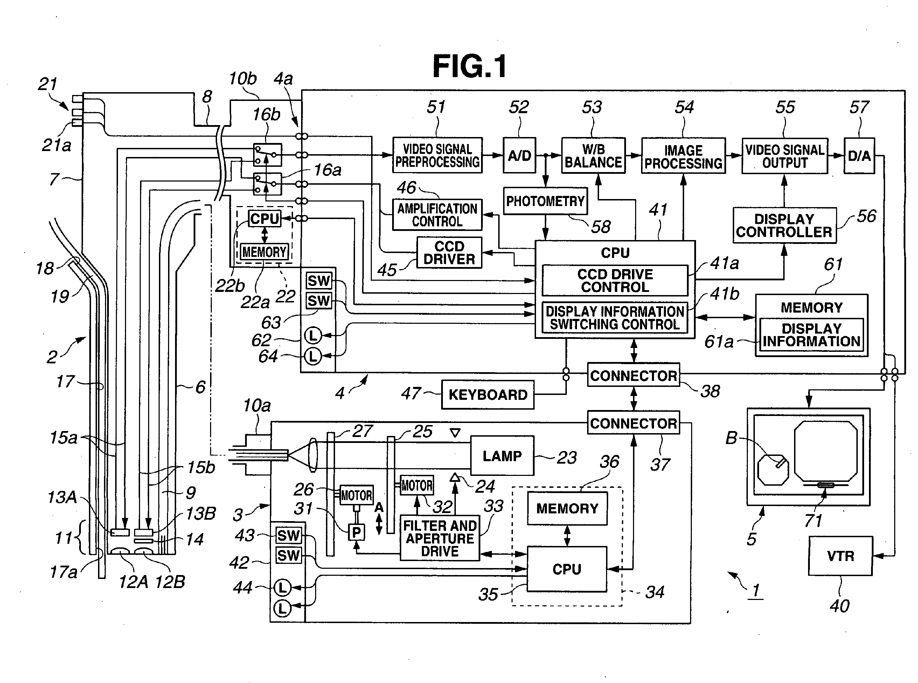

A first embodiment of the present invention will be described with reference to Figs. 1 to 5.

The present embodiment aims at providing a signal processing apparatus for an electronic endoscope and an electronic endoscope apparatus capable of appropriately displaying the information regarding the electronic endoscope corresponding to the solid-state image pickup device actually driven before and after the switching even when the driven solid-state image pickup device is switched in the electronic endoscope mounted with two different types of the solid-state image pickup devices.

As shown in Fig. 1, the electronic endoscope apparatus 1 comprising the first embodiment of the present invention comprises: an electronic endoscope 2 inserted into a body cavity and observing and treating a subject such as a diseased part, a light source 3 supplying an RGB light for normal observation and a special light for special observation to the electronic endoscope 2, a processor 4, as s signal processing apparatus for an electronic endoscope, signal-processing an endoscopic video signal picked up by the electronic endoscope 2 and generating a video signal, a monitor 5 displaying the endoscopic image corresponding to this video signal by the video signal outputted from this processor 4 being inputted, and a VTR 40, for example, as a video image recording apparatus recording the video signal in a moving image.

The monitor 5 comprising display means displays the information relating to the solid-state image pickup device actually switched together with the endoscopic image picked up by the solid-state image pickup device actually selected and used when the selection (or switching) of the observation mode is performed.

The electronic endoscope 2 has an insertion portion 6 to be inserted into the body cavity of a patient and an operation portion 7 provided at the rear end of this insertion portion 6, and from this operation portion 7, a universal cable 8 is extended. Inside this insertion portion 6, a light guide 9 transmitting an illumination light is inserted, and a light guide connector 10a at the rear end thereof is detachably connected to a light source 3.

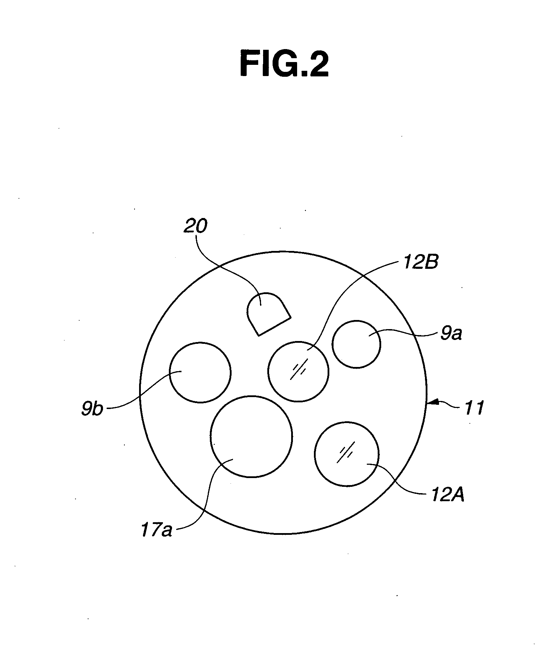

This light guide 9 transmits the illumination light (excitation light at a fluorescent light observation time in a special light observation mode) from the light source 3, and emits the illumination light to the outside from light guide distal end surfaces 9a and 9b (see Fig. 2) which are attached to an illumination window of a distal end portion 11 of the insertion portion 6, and illuminates the subject such as a diseased part (irradiates the excitation light at the fluorescent light observation time).

As shown in Fig. 2, close to the illumination window, two observation windows (image pickup windows) are provided, and the two observation windows are attached with objective lens systems 12A and 12B respectively. As shown in Fig. 1, at each image forming position of the objective lens systems 12A and 12B, first and second charge coupled devices (abbreviated as CCD) 13A and 13B as the solid-state image pickup devices are arranged.

The second CCD 13B is a high sensitivity CCD comprising an amplifying function inside the CCD device, and is used only at the fluorescent light observation mode in the special light observation mode. In contrast to this, the first CCD 13A is used at a normal observation mode (visible observation mode) observing in a visible area and at an infrared light observation mode in the special light observation mode (except for the fluorescent light observation mode) and at a narrowband light observation mode time.

As the objective lens systems 12A and 12B, a zoom optical system is formed in which portions of the lens system of the objective lens systems 12A and 12B are moved in the direction of the optical axis by unillustrated driving means so as to be changeable in zoom magnification. The electronic endoscope 2, depending on its type, includes a zoom optical system and a non-zoom optical system in the objective lens systems 12A and 12B.

As described above, the second CCD 13B is provided with an excitation light cut filter 14 in front of the CCD 13B to cut the excitation light so as to perform the fluorescent light observation at the fluorescent light observation mode.

The signal lines 15a and 15b whose one ends are respectively connected to these CCD 13A and CCD 13B are inserted into the insertion portion 6, the operation portion 7, and the universal cable 8, and the other ends reach a signal connector 10b of the end portion of the universal cable 8. This signal connector 10b is detachably connected to a signal connector receptor 4a of the processor 4. This signal connector receptor 4a forms an endoscope connecting portion to which the electronic endoscope 2 is detachably connected.

Further, inside the signal connector 10b, selector switches 16a and 16b which can be switched with interlocking are provided. By switching the signal lines 15a and 15b connected to respective CCDs 13A and 13B through the selector switches 16a and 16b, one CCD actually used for image pickup can be selected according to the observation mode.

In the present embodiment, while switching means for switching respective CCDs 13A and 13B is provided inside the electronic endoscope 2, instead of providing the switching means, the CCDs to be driven may be switched at the processor 4 side.

Further, inside the insertion portion 6, a channel 17 is provided, and this channel 17 is opened at a treatment instrument insertion port 18 in the vicinity of the front end of operation portion 7, and an operator can insert a treatment instrument 19 from this treatment instrument insertion port 18. This channel 17 is opened as a distal end opening 17a at the distal end surface of the distal end portion 11. The operator allows the distal end side of the treatment instrument 19 inserted into the channel 17 to protrude from the distal end opening 17a, and can perform a procedure such as collecting a diseased tissue or cutting off a diseased part by the treatment instrument.

Further, inside the insertion portion 6, an unillustrated air and water feed duct is provided, and a nozzle 20 of the distal end of this air and water feed duct, for example, as shown in Fig. 2, is faced to the objective lens system 12B and the objective lens system 12A at the extended end of the objective lens system 12B.
The operator can perform the removal and the like of deposits interfering with the observation field of view adhered to the external surfaces of the objective lens system 12B and the objective lens system 12A by performing the operation of an air supply or a water supply.

Further, for example, the operation portion 7 of this electronic endoscope 2 is provided with a scope switch portion 21 comprising a plurality of operation switches, and this scope switch portion 21 is provided with an observation mode selector switch 2 1 a and the like which switches or selects the observation mode.

Further, as hereinafter described, the observation mode selecting operation means such as the observation mode selector switch 21a includes the function of an observation mode selecting signal for selecting an observation mode, and at the same time, functions also as a CCD selecting signal which selects (switches) the CCD actually used corresponding to the observation mode.

Further, for example, inside the signal connector 10b of this electronic endoscope 2, a scope information storing portion 22 storing inherent information on the electronic endoscope 2 is provided.

This scope information storing portion 22 includes a memory 22a as storing means (memory means) storing the scope information, and a CPU 22b performing a processing such as storing information in this memory 22a and reading the stored information.

In the memory 22a, there are stored a plurality of data (for example, 38 pieces) of a white balance set value, and the specific data configuration of the data is, for example, in the form of [light source serial number]+[color filter type data]+[white balance set value], and the data of this type of configuration is stored in the memory 22a.

Further, the memory 22a is stored with the data and the like regarding the solid-state image pickup devices as follows in addition to the data of the white balance set value.
1) Endoscope model name
2) Endoscope serial number
3) Data on the type of observation light to which the endoscope corresponds
4) Data on the number and type of solid-state image pickup devices provided in the endoscope
5) Data on the number of pixels of each solid-state image pickup device provided in the endoscope
6) Data showing the presence or absence of adaptiveness to the optical magnified observation of the endoscope
7) Information on the treatment instrument channel of the endoscope (inner diameter of the channel, directional position for the image pickup field of view of the solid-state image pickup device, and identification color information on the applicable treatment instrument)
8) Distal end portion external diameter data of the endoscope
9) Insertion portion external diameter data of the endoscope
10) Scale data (every solid-state image pickup device) showing what mm in size a material body is visible when the endoscope corresponds to the optical magnification observation and the material body of 1 mm in size is observed at the maximum magnification

On the other hand, the light source 3 has a lamp 23 generating an illumination light including a visible light.

The illumination light emitted from this lamp 23 is incident on a band switch filter 25 through an aperture 24 arranged in the light path. The light transmitting the band switch filter 25 is incident on a rotating filter 27. The light transmitting the rotating filter 27 is converged by a converging lens system, and is incident on an incident end of the light guide 9.

The rotating filter 27 is moved, for example, by a plunger 31 in the direction (in the direction to an arrow mark shown by a symbol A in Fig. 1) orthogonal to the light path of the illumination light, together with a motor 26 which rotates this rotating filter 27 around the optical axis of the illumination light. For example, the motor 26 is attached to the end portion of the arm of the plunger 31, and by making a protruding amount of the arm variable, the rotating filter 27 and the motor 26 are moved in the direction (in the direction to the arrow mark A in Fig. 1) orthogonal to the light path of the illumination light.

The band switch filter 25 is rotatably attached to the rotating shaft of a motor 32, and this motor 32 is driven by a filter and aperture drive circuit 33. This filter and aperture drive circuit 33 drives an aperture 24, and also drives the plunger 31. The filter and aperture drive circuit 33 is controlled by a light source control circuit 34 provided to the light source 3.

This light source control circuit 34 comprises a CPU 35 as control means, and memory 36 storing inherent information on the light source 3 and the like.

The memory 36 stores the following data.
1) Serial number of light source
2) Identification information on a special light filter mounted on the light source
3) Data on the status of use of the light source (the number of use of the light source, used hours, total lightening hours of the lamp, the total number of uses and used hours of each special light filter)

The CPU 35 is connected to a connector 38 provided in the processor 4 by a signal line for communication through a connector 37 provided in the light source 3. The CPU 35 is allowed to perform two-way communications with a CPU 41 as control means provided inside the processor 4.

As hereinafter described, when the switching (or the selection) operation of the observation mode or the like is performed by the operation of the observation mode selector switch 2 1 a and the like by the user, the CPU 41 performs communications with the CPU 35 having the function of the illumination light emitting control means inside the light source 3. The CPU 41 controls so as to supply (emit) the illumination light corresponding to the observation mode to the light guide 9 of the electronic endoscope 2 through the CPU 35.

Further, the light source 3 is provided with a front panel 42. This front panel 42 is provided with a plurality of operation switches 43 to perform the switching or the selecting operation of the illumination light (also referred to as the observation light) used for the observation, and an LED 44 (abbreviated as L in Fig. 1) to advise the user by lightening or extinction whether or not the light is an observation light selectable for the case of the light source 3.

The operation switch 43 and the LED 44 are connected to the CPU 35 through the signal lines. A plurality of operation switches provided in the front panel 42 of the light source 3 are also provided with mode selector switches for switching observation mode, and when these mode selector switches are operated, the switching of the observation mode is performed.

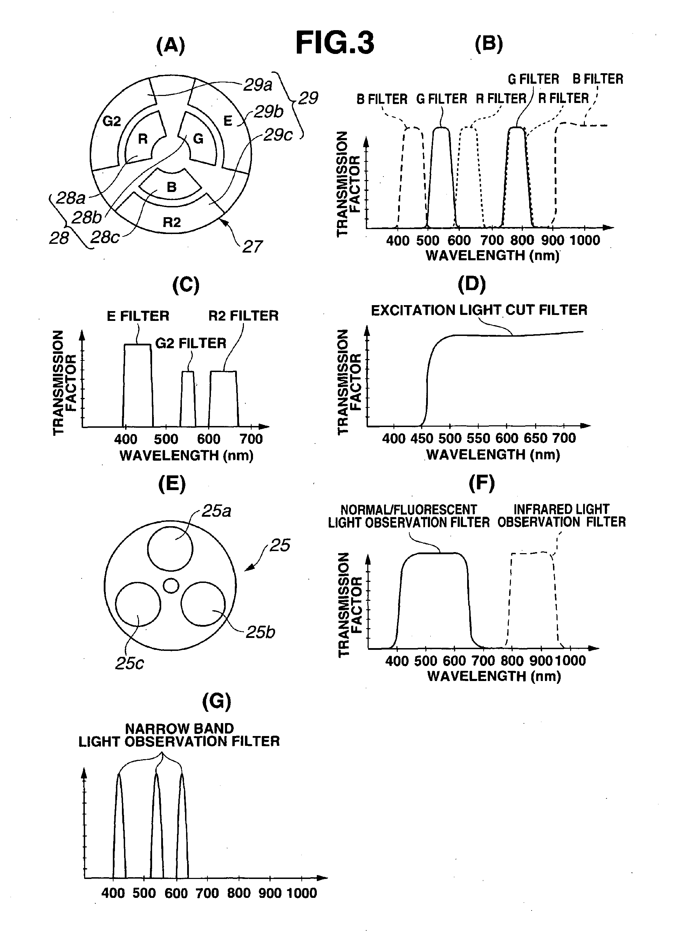

Next, referring to Fig. 3, the structure and characteristics of the rotating filter 27 and the band switch filters 25 provided in the light source 3 will be described.
Fig. 3 is an explanatory drawing regarding the structure of filters and the characteristics of each filter used in the electronic endoscope 2.

As shown in Fig. 3A, the rotating filter 27 is arranged with an RGB filter 28 for normal observation in a concentric-circular inner peripheral side, and a filter 29 for fluorescent light observation in a concentric-circular outer peripheral side. According to the observation mode, any of the filters is selected, and is inserted on the light path of the illumination light.

The RGB filter 28 for normal observation arranged inside the inner peripheral side comprises an R filter 28a, a G filter 28b, and a B filter 28c, and these filters, as shown in Fig. 3B, have transmission characteristic covering a visible wavelength area.

That is, the R filter 28a is set to transmit a red waveband of 600 mm to 700 mm, and the G filter 28b a green waveband of 500 mm to 600 mm, and the B filter 28c a blue wavelength of 400 mm to 500 mm, respectively.

Further, the RGB filter 28 is also used for infrared light observation, and hence, the R filter 28a and the G filter 28b are also set to transmit the waveband of 790 mm to 820 mm, and the B filter 28c the waveband of 900 mm to 980 mm, respectively.

The fluorescent light observation filter 29 for fluorescent light observation arranged in the outer peripheral side comprises a G2 filter 29a, an E filter 29b, and an R2 filter 29c, and each filter has the transmission characteristic as shown in Fig. 3C.

That is, the G2 filter 29a is set to transmit the waveband of 540 mm to 560 mm, the E filter 29b the waveband of 400 mm to 470 mm, and the R2 filter 29c the waveband of 600 mm to 660 mm, respectively. In the meantime, the transmission characteristic of the G2 filter 29a and the R2 filter 29c are set to a low level, and by synthesizing the green and red color signals (hereinafter, referred to as G2 signal and R2 signal, respectively) picked up under the illumination light of these narrow bands and the fluorescent light signal, a color display can be made for fluorescent light observation.

Further, as shown in Fig. 3E, the band switch filter 25 is arranged with a filter 25a for normal/fluorescent light observation, a filter 25b for narrow band light observation, and a filter 25c for infrared light observation on a concentric-circle, and according to the observation mode, any of the filters is selected, and is inserted on the light path of the illumination light.

As shown in Fig. 3F, the filter 25a for normal/fluorescent light observation is set to transmit the waveband in the vicinity of 400 nm to 660 nm, the filter 25c for infrared light observation is set to transmit the waveband in the vicinity of 780 nm to 950 nm. Further, the filter 25b for narrow band light observation comprises a filter with triplet peaks, and as shown in Fig. 3G, is set to transmit three discreet wavebands of the vicinity of 400 nm to 430 nm, the vicinity of 530 nm to 550 nm, and the vicinity of 600 nm to 630 nm.

In an electronic endoscope 1 comprising the present embodiment, by restricting the wavebands of the illumination light, the subject can be observed by a total of four type observation modes i.e, the normal observation and three types of the narrow band light observation corresponding to the special light observation, the infrared light observation, and the fluorescent light observation. More specifically, in the case of the normal observation, the image pickup is performed under a frame sequential light of the visible light area by R. G. B, and the normal endoscopic image is generated for the picked up signal.

In contrast to this, in the case of the narrow band light observation, the infrared light observation, and the fluorescent light observation, the band restriction and the like of the wavelength used for the illumination or the image-pickup are performed, thereby generating the corresponding image, respectively. Further, in the present embodiment, particularly in the case of the fluorescent light observation, the fluorescent light strength is extremely weak comparing with other observation times, and therefore, a high sensitivity CCD 13B comprising a signal amplification (signal multiplication) function inside the above described CCD device is selected to be used.

These observation modes are set by operating the selection operating means such as the observation mode selector switch 21 a and the like by the user. When the observation mode selector switch 21a is operated, an instruction signal is outputted to the CPU 41 comprising the control means inside the processor 4.

The CPU 41 sends the instruction signal (more specifically a mode selecting signal) of the observation model selector switch 21a to the CPU 35 of the light source 3. The CPU 35 controls the rotation amount (rotation angle) of the plunger 31 and the motor 32 through the filter and aperture drive circuit 33, so that the filter arranged in the illumination light path of the lamp 23 is switched to the RGB filter 28 or the filter 29 for fluorescent light observation according to the observation mode instructed, and the band switch filter 25 is selected and controlled.

Specifically, when the observation mode is set to the normal observation mode, the narrow band light observation mode, and the infrared light observation mode, the RGB filter 28 arranged in the inner peripheral side of the rotating filter 27 is inserted on the light path of the illumination light. When the fluorescent light observation mode is set, the filter 29 for fluorescent light observation arranged in the outer peripheral side of the rotating filter 27 is inserted on the light path of the illumination light.

Further, when the observation mode is set to the normal observation mode and the fluorescent light observation mode, the filter 25a for normal/fluorescent light observation is inserted on the light path of the illumination light. When the observation mode is set to the narrow band light observation mode, the filter 25b for narrow band light observation is inserted on the light path of the illumination light. When the observation mode is set to the infrared light observation mode, the filter 25c for infrared light observation is inserted on the light path of the illumination light.

That is, in the normal observation mode, the illumination light emitted from the lamp 23 transmits the filter 25a for normal/fluorescent light observation having the characteristics shown in Fig. 3F and the RGB filter 28 having the characteristics shown in Fig. 3B, so that the light only of the waveband of Red, Green, and Blue is filtered, and is emitted sequentially from the light source 3 to the light guide 9.

Further, in the narrow band light observation mode, the illumination light emitted from the lamp 23 transmits the filter 25b for narrow band light observation having the characteristics shown in Fig. 3G and the RGB filter 28 having the characteristics shown in Fig. 3B, so that the lights only of the wavebands of 600 nm to 630 nm, 530 nm to 560 nm, 400 nm to 430 nm are filtered, and are emitted sequentially from the light source 3 to the light guide 9.

Further, in the infrared light observation mode, the illumination light emitted from the lamp 23 transmits the filter 25c for infrared light observation having the characteristics shown in Fig. 3F and the RGB filter 28 having the characteristics shown in Fig. 3B, so that the lights only of the waveband of 790 nm to 820 nm, 790 nm to 820 nm, and 900 nm to 980 nm are filtered, and are emitted sequentially from the light source 3 to the light guide 9.

Further, in the fluorescent light observation mode, the illumination light emitted from the lamp 23 transmits the filter 25a for normal/fluorescent light observation having the characteristics shown in Fig. 3F and the filter 29 for fluorescent light observation having the characteristics shown in Fig. 3C, so that the lights only of the waveband of 540 nm to 560 nm, 390 nm to 450 nm, and 600 nm to 620 nm are filtered, and are emitted sequentially from the light source 3 to the light guide 9. Here, the light of the waveband of 390 nm to 450 nm is used as the excitation light to excite an auto-fluorescence light from a living tissue.

The illumination light incident on the light guide 9, as shown in Fig. 3, is emitted from the distal end surfaces 9a and 9b of the light guide 9, and is irradiated on the subject such as a test subject site. In the normal observation mode, the frame sequential illumination light of R.G.B. is irradiated on the subject, and in the fluorescent light observation mode, the frame sequential illumination light of G2, E, and R2 is irradiated on the subject (the illumination light of E is used as the excitation light).

In the fluorescent light observation mode, the CCD 13B is used, and on the light path between this CCD 13B and the objective lens system 12B, the excitation light cut filter 14 is arranged, and this filter blocks the excitation light of 390 nm to 450 nm among the reflected lights from the subject, thereby to extract the fluorescence light.

The excitation light cut filter 14, as shown in Fig. 3D, is set to transmit the waveband of 470 nm or more, and is set so as not to overlap with the transmission characteristic of the E filter 29b.

The frame sequential illumination light is irradiated, and a scattered light, a reflected light or fluorescence light are generated from the subject. These lights transmit the excitation light cut filter 14, and are image-formed on the photoelectric conversion surface of the CCD 13B by the objective lens system 12B, and are photoelectric-converted in the CCD 13B.

In the present embodiment, the image signal corresponding to the illumination light which has transmitted each filter of the rotating filter 27 is sequentially outputted from the CCD 13A or CCD 13B to the processor 4 in time sequence.

The image signal (pickup image signal) outputted in time sequence becomes the color signal of R. G. B. in the normal observation mode, a G2 signal picked up under the illumination light of G2, a fluorescent light signal picked up under the excitation light of E, and a signal picked up under of the illumination light of R2 signal in the fluorescent observation mode. Further, in the narrow band light observation mode and the fluorescent light observation mode, the image signal becomes a signal according to the sequence of each illumination light.

Next, with reference to Fig. 1, the internal structure of the processor 4 will be described. Inside the processor 4, a CCD drive circuit 45 driving the CCDs 13A and 13B is provided. The CPU 41 controls the CCD drive circuit 45 so that the CCD according to the selection (switching) of the observation mode is driven. That is, the CPU 41 has a CCD drive control function 41a selecting and controlling the CCD to drive.

More specifically, the CCDs 13A and 13B are CCDs different in type, and at the same time, the number of pixels is different. Hence, when the observation mode is selected by the user, the CPU 41 controls the CCD drive circuit 45 in such a manner that the CCD drive signal driving the CCD corresponding to the observation is outputted. Further, the CPU 41 controls also the switching of the selector switches 16a and 16b according to the selection of the observation mode.

Further, inside the processor 4, an amplification factor control circuit 46 is provided, and during the period in which the fluorescent light observation is selected and the fluorescent light image pickup is actually performed, the amplification factor control circuit 46 outputs an amplification factor control signal together with the CCD drive signal to the CCD 13B from the CCD drive circuit 45.

The CPU 41 sends a control signal to this amplification factor control circuit 46 during the period in which the fluorescent light image pickup is actually performed (during the period in which the excitation light is irradiated by the E filter 29b so as to perform the fluorescent light image pickup); and, for example, so as to output an amplification factor control signal set to the standard amplification factor corresponding to the pre-set fluorescent light observation.

This amplification factor control signal is superposed with a CCD drive signal and is applied to the CCD 13B, and the signal photoelectric-converted in the CCD 13B device is multiplied by the amplification factor control signal, and the amplified signal is outputted from the CCD 13B.

The user, for example, transmits an instruction signal for setting to an arbitrary amplification factor from a keyboard 47 to the CPU 41, thereby making it possible to change the amplification factor. Further, the instruction signal by the operation switch increasing and decreasing the amplification factor allotted to a plurality of scope switch portions 21 is transmitted to the CPU 41, thereby making it possible to change the amplification factor. In this case also, by these instruction signals, the CPU 41 sends the corresponding control signals to the amplification factor control circuit 46 so as to set to the instructed amplification factor.

The CCD 13A or the CCD 13B, with the CCD drive signal applied, outputs a photoelectric-converted image pickup signal. This image pickup signal is inputted into a video signal preprocessing circuit 51 inside the processor 4, and is subjected to a CDS processing and the like by this video signal preprocessing circuit 51.

The output signal of the video signal preprocessing circuit 51 is inputted to an A/D converting circuit 52 converting an analogue signal into a digital signal. The video signal converted into the digital signal is inputted to a white balance circuit (abbreviated as W/B balance in Fig. 1) 53 performing a white balance processing. The output signal of this white balance circuit 53 is, for example, inputted to an image processing circuit 54 performing an image processing such as structural emphasis and chromatic emphasis.

The output signal of this image processing circuit 54 is inputted to a video signal output circuit 55 synthesizing this output signal and the video signals corresponding to various types of images generated by a display controller 56, thereby to be outputted. The output signal of this video signal output circuit 55 is inputted to a D/A converting circuit 57, and is converted into an analogue video signal and outputted to a monitor 5.

Further, the output signal of the A/D converting circuit 52 is inputted to a photometric circuit 58 measuring brightness on the image to perform an automatic control of the amount of the illumination light, and is subjected to photometry by this photometric circuit 58. As a mode to perform the photometry, there are a peak photometry to detect a peak of brightness of the image, an average photometry to detect an average brightness, and an auto photometry to detect brightness in the center vicinity.

This photometric circuit 58 or the CPU 41 inputted with a signal treated with photometry compares the signal treated with photometry with a reference value (brightness) intended to be set, and generates a modulated light signal so as to narrow down the comparison difference. This modulated light signal is sent to the CPU 35 inside the light source 3 through the connectors 38 and 37, and this CPU 35 adjusts an aperture amount of the aperture 24 through the filter and aperture drive circuit 33 so as to automatically modulate the signal to become adequate brightness equivalent to the reference value.

Further, inside the processor 4, a memory 61 storing various types of information is provided, and the CPU 41, referring to the display information 61a and the like stored in this memory 61, performs the switching control of the display information to be displayed in the monitor 5. That is, the CPU 41 has the function of the display information switching control 41b. The switching of the display information displayed in the monitor 5 will be described later.

Further, inside the processor 4, the memory 61 storing various pieces of information is provided, and the CPU 41, by referring to the information stored in this memory 61, performs a control so as to advise whether or not the functions allotted to the plurality of operation switches 63 of the front panel (operational panel) 62 provided in the front surface of the processor 4 are in a state of being executable by the display of lighting /extinction of the LED 64.

The operation switch 63 and the LED 64 are connected to the CPU 41 through the signal lines. The CPU 41 performs an operation control of the image processing circuit 54 corresponding to the operation of the operation switch 63.

This CPU 41 controls the white balance circuit 53, the display controller 56, and the like in addition to the image processing circuit 54.

The processor 4 in the present embodiment comprising such a configuration includes the CPU 41 reading the information regarding the types or the like of the electronic endoscope 2 detachably connected to this processor 4 from the memory 22a provided in the electronic endoscope 2, and this CPU 41 temporarily stores the read information in the memory 61. Further, when the electronic endoscope 2 is built-in with the plurality of CCDs, the CPU 41 has the function of a switch determination portion to perform a determination as to whether or not the CCD to be driven is switched according to the selection of the observation mode by the user.

When the CCD to be driven is switched, the CPU 41 performs a switch control of the display information in such a manner that the display information displayed in the monitor 5 according to the switching is made to correspond to the CCD. In the meantime, at the initial state by turning the power on, the CPU 41 or the CPU 35 sets the illumination light of the normal observation mode to a state of being supplied to the endoscope (electronic endoscope or a fiber scope), and at the same time, sets the signal processing system of the processor 4 also to a state corresponding to the normal observation mode.

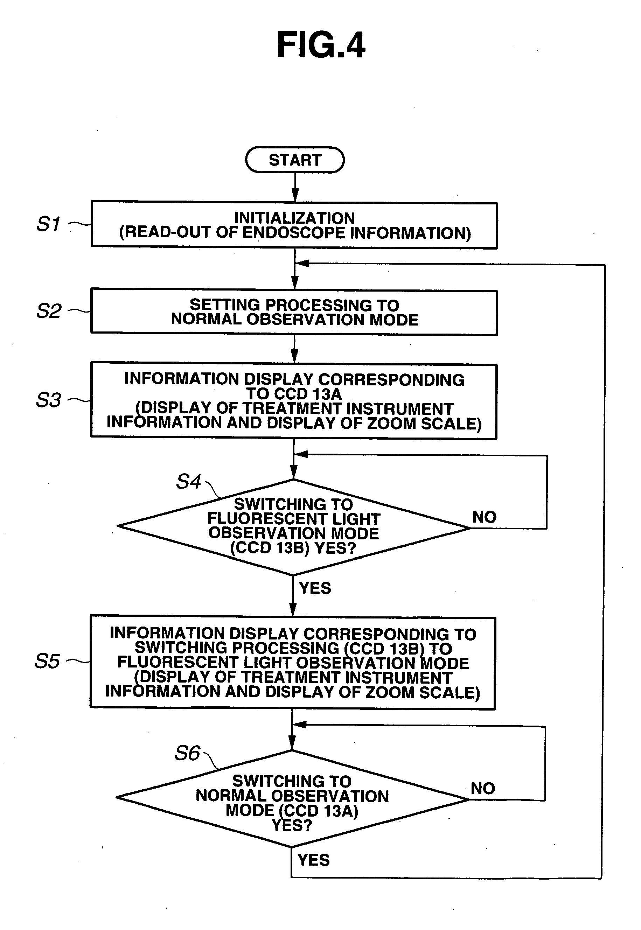

Next, the operation of displaying the information relating to the electronic endoscope 2 connected to the electronic endoscope apparatus 1 in the present embodiment thus configured will be described with reference to Fig. 4.

When the power of the light source 3 and the processor 4 are turned on in a state in which the electronic endoscope is connected to the light source 3 and the processor 4 as shown in Fig. 1, the light source 3 and the processor 4 are put into an operating state.

Then, the CPU 35 of the light source 3 and the CPU 41 of the processor 4, for example, read the programs stored in the internal ROM or the like, and as shown in step S1, perform the processing of initialization.

In the processing of this initialization, for example, the CPU 41, to determine the type and the like of the electronic endoscope 2 connected to the processor 4, reads the information stored in the memory 22a provided in the interior of the electronic endoscope 2 connected to the processor 4, thereby to perform the processing of reading the endoscope information.

As shown in step S2, the CPU 41 determines from the information read from the memory 22a that this electronic endoscope 2 is an electronic endoscope comprising two CCDs 13A and 13B, and performs a control of setting the endoscope to a state of the normal observation mode.

Hence, the CPU 41 controls the selector switches 16a and 16b to a state of driving the CCD 13A, and at the same time, performs communications with the CPU 35 of the light source 3, and under the control of the CPU 35, puts the illumination light of the light source 3 in a state of supplying the illumination light of the normal observation mode to the electronic endoscope 2.

Further, in the next step S3, the CPU 41 performs a control of the display information to display in the monitor 5 the information relating to the electronic endoscope 2, more specifically, the information relating to the electronic endoscope 2 in a state corresponding to the CCD 13A actually driven by that electronic endoscope 2 or performs a switch control of the display information.

In this case, the control processing of the display information to be displayed on the monitor 5 is performed regarding the information on the inner diameter of the treatment instrument channel 17 of this electronic endoscope 2 connected to the processor 4 and the treatment instrument information on the treatment instrument direction or the like in which the treatment instrument appears in the image pickup range (field of view range) in the case of the CCD 13A (specifically the CCD 13A at the normal observation time) in its operating state and the current zoom scale by the objective lens system 12A which forms an image in the CCD 13A and the like.

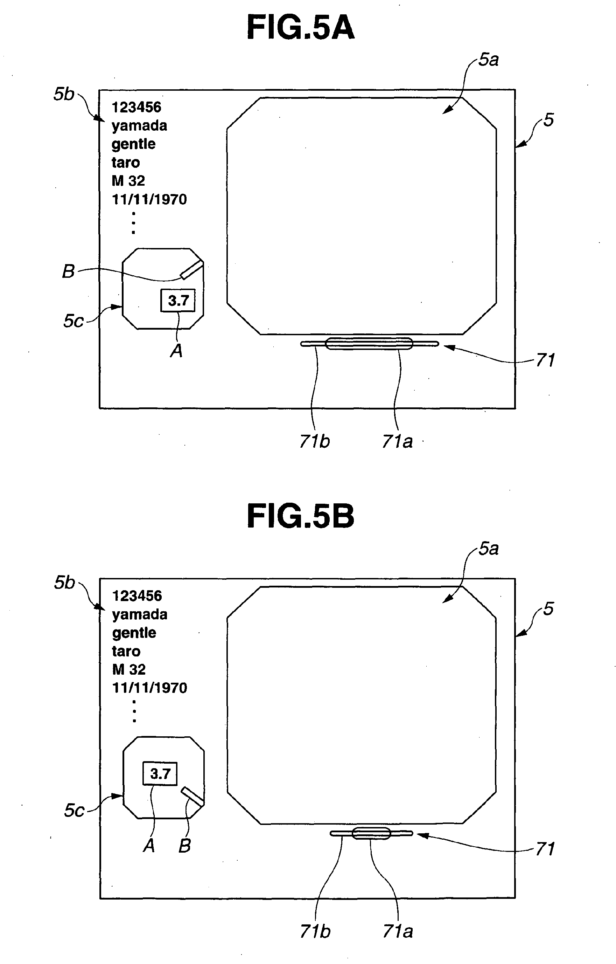

The display example by the monitor 5 in this case is shown in Fig. 5A. As shown in Fig. 5A, approximately in the center vicinity (slightly close to the right side upper portion side) in the display surface of the monitor 5, there is an endoscope image display area 5a which displays an endoscope image picked up by the CCD 13A, and at the left side of the display surface, there is a patient information and the like display area 5b in which the patient information and the like are displayed.

In the part of this patient information and the like display area 5b, there is provided a treatment instrument information display area 5c which reduces the size of the endoscope image display area 5a. In this treatment instrument information display area 5c, the treatment instrument information comprising an information A on the inner diameter (3.7 indicating that it is 3.7 mm in Fig. 5A) of the treatment instrument channel 17 of this electronic endoscope 2 and a treatment instrument directional information B in which the treatment instrument appears in the image pickup range is displayed under the control of the CPU 41.

Further, at the bottom of this endoscope image display area 5a, the zoom scale 71, which shows an approximate scale in the endoscope image which is picked up by the CCD 13A currently being driven by using the objective lens system 12A and displayed in the endoscope image display area 5a, is displayed under the control of CPU 41.

This zoom scale 71 comprises a far-point side scale 71A showing what mm it appears on the screen when a material body of 1 mm in size is observed, for example, at the maximum expansion time and a near-point side point scale 71 b, and both of the scales 71a and 71b are displayed under the control of the CPU 41 based on the information on the optical expansion observation stored in the memory 22a.

Describing furthermore, when a predetermined length at the maximum expansion time is actually displayed on the screen, the zoom scale 71 represents how long the length becomes and in the present embodiment, the zoom scale 71 comprises two of the near-point side scale 71a (the range also at the front most side) of the depth (the range coming into focus) at the maximum expansion time and the far-point side scale 71b (at the rear most side). In this manner, at the bottom of the endoscope image display area 5a, a scale capable of estimating (measuring) the length in the mage is displayed, and this enables the user, by referring to the scale, to grasp a size of the image portion such as a diseased part, thereby making it easy to undertake a diagnosis.

The image of the treatment instrument information display area 5c and the display image of the zoom scale 71 are generated by the display controller 56 under the control of the CPU 41. The video signal of the generated display image is mixed (and synthesized) with the video signal of the endoscope signal picked up by the CCD 13A in the video signal output circuit 55, and the synthesized image is displayed in the monitor 5 as shown in Fig. 5A. As such, on the display surface of the monitor 5 the information regarding the electronic endoscope 2 actually connected to the processor 4, and more specifically, the information (relating to the electronic endoscope 2) corresponding to the CCD 13A actually used is displayed.

After that, at the next Step S4, the CPU 41 performs a determination as to whether or not a switch instruction to the fluorescent light observation mode in the special light observation mode, in other words, the switch instruction to the second CCD 13B is issued. The CPU 41 keeps its display state until receiving the switch instruction. However, when the instruction operation of the zoom change is performed, the CPU 41 performs the display of the zoon scale 71 corresponding to this zoom change.

The operator, in the normal observation mode, observes the diseased part and the like, and further, when the operator wants to observe the diseased part by the fluorescent observation mode, the operator performs the operation selecting the fluorescent observation mode, for example, by the observation mode selector switch 21a.

When the operation of the switch instruction to the fluorescent light observation mode is performed, as shown at step S5, the CPU 41 and the like execute a switch processing to perform the illumination and the like corresponding to the fluorescent light observation mode. Specifically, the CPU 41 sends the instruction signal to the CPU 35 of the light source 3 to switch the normal observation mode to the fluorescent observation mode.

The light source 3 is put into a state of supplying the illumination light corresponding to the fluorescent light observation mode to the electronic endoscope 2. Further, the CPU 41 switches the selector switches 16a and 16b, and moreover, performs a control such that the CCD drive signal outputted from the CCD drive circuit 45 becomes a signal to drive the CCD 13B, and at the same time, controls the amplification factor control circuit 46.

During the period of performing the fluorescent image pickup (that is, the period of illuminating the excitation light passing through the E filter of Fig. 3A and performing the fluorescent light image pickup), the CCD drive signal is superposed with the amplification factor control signal from the amplification factor control circuit 46, and is applied to the CCD 13B. As a result, the signal level of the fluorescent light pickup image signal performing the fluorescent light image pickup is made close to the signal level picked up by other reflected light.

Further, the CPU 41 performs a display control processing to display in the monitor 5 the information regarding the electronic endoscope 2 corresponding to the switched and actually driven CCD 13B in conjunction with the switching of the CCD driven from the CCD 13A to the CCD 13B (performs a switch control of the displayed information if expressed in terms of before and after the switching).

Specifically, the CPU 41 performs a display processing to display in the monitor 5 the treatment instrument information such as the treatment instrument directional information in which the treatment instrument in the case of this CCD 13B appears in the image pickup range and the current zoom scale and the like when this CCD 13B is used.

As shown in Fig. 5B, at this fluorescent light observation mode time, the display information is changed to the display content of the information relating to the electronic endoscope 2 corresponding to the case of the CCD 13B actually being used for the image pickup.

In other words, the display content is changed corresponding to the switching from the CCD 13A to the CCD 13B in the case of FIG. 5A. Specifically, the treatment instrument information and the current zoom scale 71 and the like by the objective lens system 12B and the CCD 13B are displayed in the monitor 5.

In this case, the information A on the inner diameter of the treatment instrument channel 17 of the electronic endoscope 2 is the same as that of Fig. 5A, whereas the treatment instrument directional information B, in which the treatment instrument appears in the image pickup range, is different from that of Fig. 5A, and becomes the display corresponding to the CCD 13B. In other words, in Fig. 5A, the treatment instrument directional information B is directed to the center from the right upper side, whereas in Fig. 5B, the treatment instrument directional information B is directed to the center from the right bottom side.

Further, the zoom scale 71 displayed directly below the endoscope image is changed to a value by the objective lens system 12B forming an image in the CCD 13B currently being used for the image pickup. In the example of Fig. 5B, the zoom scale 71 which is smaller than the case of the objective lens system 12A is displayed.

At step S6 after step S5, the CPU 41 is put in a state of waiting for the switching to the normal observation mode (CCD 13A). When the switching to the normal observation mode is performed, the operation returns to step S2.

According to the present embodiment, in the case of the electronic endoscope 2 mounting two CCDs 13A and 13B, when the CCD is used by switching one CCD to the other CCD, the information relating to the electronic endoscope 2 corresponding to the switched CCD corresponding to the switching is appropriately displayed.

Consequently, the treatment by the treatment instrument is easy to perform, and at the same time, even when the diseased part and the like are enlarged and observed, its size is easy to grasp, and a diagnosis is easy to undertake. In other words, when the operator performs the endoscopy and the treatment by the treatment instrument, these can be performed in a user friendly state. That is, a good operability can be secured.

In the description made so far, when the processor 4 is connected with the electronic endoscope 2, the processor 4 reads the information regarding the electronic endoscope 2 stored in the memory 22a built-in the electronic endoscope 2, and displays the information in the monitor 5 by utilizing the information.

In this case, the memory 22a is stored with inherent identification information on the electronic endoscope 2, and the information regarding the electronic endoscope 2 corresponding to the identification information may be stored in the processor 4 side.

In the description made so far, for example, the CCD 13B has been described with the case of the CCD comprising the signal amplifying function inside the CCD device, but the same can be applied even by the CCD whose number of pixels or the like are different from the CCD 13A. Further, the CCD 13A may comprise the signal amplification function inside the CCD device like the CCD 13B.

In the description made so far, a description has been made by the case of the selection (switching) of one CCD driven according to the selection of the observation mode, but when taking into consideration the case where a plurality of CCDs can be driven by the common driving signal, the plurality of CCDs may be always driven by the common drive signal. Further, according to the selection of observation mode, one from the plurality of CCD output signals may be selected.

When the plurality of CCDs are driven by the common drive signal, a CCD drive circuit which generates a drive signal to drive the CCD having a larger number of pixels may be adopted.

According to the present invention, before and after the switching of the solid-state image pickup devices, the information regarding the electronic endoscope outputted to the display means can be corresponded to the solid-state image pickup device to be switched, thereby improving the operability.

### Industrial Applicability

According to the selecting operation and the like of the observation mode, the corresponding CCD is used by selecting from two CCDs, and the information regarding the electronic endoscope corresponding to the CCD to be used is displayed, so that the endoscopy and the treatment and the like by using the treatment instrument can be performed in a more user friendly manner.

## Claims

1. A signal processing apparatus for an electronic endoscope comprising:
an endoscope connecting portion connected with an electronic endoscope provided with at least a plurality of solid state image pickup devices;
a selecting operation portion for performing an operation to generate a selecting signal to perform the selection of at lease one output signal of the plurality of solid state image pickup devices provided in the electronic endoscope connected to the endoscope connecting portion;
a signal processing circuit performing signal processing for the output signal of one solid-state image pickup device selected based on the selecting signal and generating a video signal; and
an information switch control portion performing the switching of the information to be displayed in the display device regarding the electronic endoscope corresponding to one selected solid-state image pick up device whose output signal is selected.

2. The signal processing apparatus for an electronic endoscope according to claim 1, comprising a drive signal generating circuit applying a drive signal to one solid-state image pickup device selected based on the selecting signal.

3. The signal processing apparatus for an electronic endoscope according to claim 1, wherein the information regarding the electronic endoscope is the solid-state image pickup device related information relating to each of the plurality of solid-state image pickup devices.

4. The signal processing apparatus for an electronic endoscope according to claim 1, wherein the information regarding the electronic endoscope is an information on the scale for estimating a length when each image picked up by each of the plurality of solid-state image pickup devices is displayed in a display device.

5. The signal processing apparatus for an electronic endoscope according to claim 3, wherein the information regarding the electronic endoscope is a treatment instrument related information regarding the direction in which the treatment instrument protruded from the distal end opening of the treatment instrument channel arranged in the peripheral portion of the plurality of solid-state image pickup devices arranged at the distal end portion of the electronic endoscope appears in the image pickup range of the plurality of solid-state image pickup devices.

6. The signal processing apparatus for an electronic endoscope according to claim 5, wherein the treatment instrument related information includes the information on the inner diameter of the treatment instrument channel.

7. The signal processing apparatus for an electronic endoscope according to claim 1, comprising a reading portion reading the stored information stored in a information storing portion inside the electronic endoscope from the electronic endoscope connected to the endoscope connection portion.

8. The signal processing apparatus for an electronic endoscope according to claim 1, wherein the signal processing apparatus for an electronic endoscope is electrically connected to a light source selectively emitting a plurality of different types of illumination lights including a first illumination light of a visible area corresponding to a first observation mode to observe in the visible area and a second illumination light corresponding to a second observation mode different at least in wavelength from the first illumination light.

9. The electronic endoscope apparatus according to claim 8, wherein the selecting operation portion is an observation mode selecting portion which generates a mode selecting signal selecting first or second observation mode as a selecting signal.

10. The electronic endoscope apparatus according to claim 9, wherein the light source selectively emits the first or second illumination light corresponding to the first or second observation mode by the mode selecting signal.

11. The electronic endoscope apparatus according to claim 9, wherein a switching determination is performed as to whether or not the switching for selecting an output signal of another solid-state image pickup device from one solid-state image pickup device selected before the generation of the mode selecting signal by the generation of the mode selecting signal.

12. An electronic endoscope, comprising:
an electronic endoscope provided with a plurality of solid-state image pickup devices;
a selecting operation portion for performing an operation to generate a selecting signal to perform the selection of at lease one output signal of the plurality of solid state image pickup devices provided in the electronic endoscope;
a signal processing circuit performing signal processing for the output signal of one solid-state image pickup device selected based on the selecting signal and generating a video signal; and
an information switch control portion performing the switching of the information to be displayed in the display device regarding the electronic endoscope corresponding to one selected solid-state image pick up device whose output signal is selected.

13. The electronic endoscope according to claim 12, comprising a drive signal generating circuit for applying a drive signal to one solid-state image pickup device selected based on the selecting signal.

14. The electronic endoscope according to claim 12, wherein the information regarding the electronic endoscope is the solid-state image pickup device related information relating to the plurality of solid-state image pickup devices respectively.

15. The electronic endoscope according to claim 12, wherein the information regarding the electronic endoscope is an information on the scale for estimating a length when each image picked up by each of the plurality of solid-state image pickup devices is displayed in the display device.

16. The electronic endoscope according to claim 12, wherein at least one in the plurality of solid-state image pickup devices is a solid-state image pickup device built-in with a signal amplifying function.

17. The electronic endoscope according to claim 12, wherein the electronic endoscope has an information storing portion for storing the endoscope information regarding the electronic endoscope.

18. The electronic endoscope according to claim 17, wherein the information switch control portion generates the information regarding the electronic endoscope by using the endoscope information read from the information storing portion.

19. The electronic endoscope according to claim 12, wherein the electronic endoscope further includes a light source for selectively emitting the plurality of types of illumination lights including a first illumination light of a visible area corresponding to a first observation mode for observing in the visible area and a second illumination light corresponding to a second observation mode different at least in wavelength from the first illumination light.

20. The electronic endoscope according to claim 19, wherein the selecting operation portion is an observation mode selecting portion for generating the mode selecting signal to select the first or second observation mode as the selecting signal, and the light source selectively emits the first or second illumination light corresponding to the first or second observation mode by the mode selecting signal.

21. The electronic endoscope according to claim 19, wherein a switching determination is performed as to whether or not the switching for selecting an output signal of another solid-state image pickup device from one solid-state image pickup device selected before the generation of the mode selecting signal by the generation of the mode selecting signal.
